# EUROPEAN PATENT SPECIFICATION

(11) **EP 4 313 010 B1**
(45) Date of publication and mention of the grant of the patent: **19.02.2025**
(21) Application number: 22712587.9
(22) Date of filing: 14.03.2022
(51) Int. Cl.: A61K 31/05, A61K 31/09, A61P 31/14

(54) **COMPOUNDS FOR USE IN THE TREATMENT AND PREVENTION OF COVID- 19**
VERBINDUNGEN ZUR VERWENDUNG IN DER BEHANDLUNG UND PRÄVENTION VON COVID-19
COMPOSÉS À UTILISER DANS LE TRAITEMENT OU LA PRÉVENTION DE LA COVID-19

(30) Priority: 25.03.2021 EP 21164852; 25.03.2021 EP 21164854
(43) Date of publication of application: 07.02.2024
(73) Proprietor: Szekeres, Thomas, 1010 Wien (AT); Jäger, Walter, 3021 Pressbaum (AT)
(72) Inventor: Szekeres, Thomas, 1010 Wien (AT); Jäger, Walter, 3021 Pressbaum (AT)
(74) Representative: SONN Patentanwälte GmbH & Co KG
(86) International application number: PCT/EP2022/056429
(87) International publication number: WO 2022/200086

(56) References cited:
- CN-A- 111 228 343
- WAHEDI HUSSAIN MUSTATAB ET AL: "Stilbene-based natural compounds as promising drug candidates against COVID-19", JOURNAL OF BIOMOLECULAR STRUCTURE & DYNAMICS, 12 May 2020 (2020-05-12), US, pages 1 - 10, XP055813453, ISSN: 0739-1102, Retrieved from the Internet <URL:https://www.tandfonline.com/doi/pdf/10.1080/07391102.2020.1762743?needAccess=true> DOI: 10.1080/07391102.2020.1762743
- ZHANG QI ET AL: "Heparan sulfate assists SARS-CoV-2 in cell entry and can be targeted by approved drugs in vitro", CELL DISCOVERY, vol. 6, no. 1, 4 November 2020 (2020-11-04), pages 1 - 14, XP055834625, Retrieved from the Internet <URL:https://www.nature.com/articles/s41421-020-00222-5.pdf> DOI: 10.1038/s41421-020-00222-5
- HAN YING-SHAN ET AL: "A resveratrol analog termed 3,3?,4,4?,5,5?-hexahydroxy-trans-stilbene is a potent HIV-1 inhibitor", JOURNAL OF MEDICAL VIROLOGY, vol. 87, no. 12, 1 December 2015 (2015-12-01), US, pages 2054 - 2060, XP055834716, ISSN: 0146-6615, DOI: 10.1002/jmv.24271

## Description

The present invention relates to the treatment and prevention of COVID-19.

In December 2019 starting in Wuhan/China a novel corona virus (SARS-CoV-2) caused a pandemic which will last until an effective and safe vaccine can be provided or causal drug treatment of the disease can help to avoid severe and fatal cases of the illness. By now millions of infected persons with increasing number all over the world are waiting for an effective treatment of the disease.

To date, no specific drug in controlling this disease has been identified. Developing the new treatment is usually time consuming, therefore using the repurposing broad-spectrum antiviral drugs was generally regarded as an effective strategy to respond immediately. Accordingly, the effects of already known antiviral compounds were heavily investigated.

Especially "repurposing" naturally occurring or previously developed synthetic antiviral compounds with a broad spectrum of biochemical mechanisms for COVID-19 were viewed as a promising strategy to combat this pandemic.

Substances like griffithsin, nafamostat (targeting cell entry), disulfiram, lopinavir/ritonavir, danoprevir, nelfinavir (targeting the SARS-CoV-2 protease), favipiravir, ribavirin, penciclovir, remdesivir, galidesivir (targeting the RNAdependent RNA polymerase (RdRp)) have been discussed as promising candidates, yet without providing a specific treatment efficient to SARS-CoV-2 in patients with COVID-19 infection (Ghanbari et al., Fut. Microbiol. 15 (2020), 1747-1758; Harrison, Nat. Biotechnol. 38 (2020), 379-381).

Phenolic compounds are uniformly dispersed phytochemicals contained and abundant in any tissue of most plant families around the world, especially in fruits and vegetables that are part of the plant. Phenolic compounds are classified based on their chemical structures into phenolic acids, flavonoids, tannins, coumarins, lignans, quinones, stilbenes, and curcuminoids. Phenolic compounds are synthesized through the shikimic acid pathway in plants as secondary metabolites are generally involved in plant adaptation to environmental stress conditions. Phenolic and flavonoids compounds are secondary metabolites of the plant that possess an aromatic ring with at least one hydroxyl group. Among the chemically diverse natural therapeutic agents, flavonoid and phenolic compounds were speculated to be most promising active compounds against SARS-CoV-2, due to their excellent pharmacokinetic properties.

Phenolic related compounds have been reported to possess numerous biological activities such as antioxidants, anti-cancer, anti-inflammatory, antibacterial, cardioprotective and immune system promoting activities. Several studies have shown that phenol and flavonoids related compounds from medicinal plants increases human health and boost human immune power. Natural polyphenolic compounds are mainly derived from plant origins. These phenolic and flavonoid class of compounds possess antiviral activities against a number of viruses such as rhinoviruses, hepatitis C virus, HIV, yellow fever, herpes simplex virus, and influenza viruses. Nowadays, pharmacology companies manufacture potential drug molecules in a short period of time with the aid of bioinformatics tools and applications.

With such in silico predictions and biochemical in vitro binding assays to SARS-CoV-2 components, a number of natural compounds, which have shown previously inhibitory effects against other pathogens, such as polyphenols, were suggested to be used in the treatment of COVID-19 patients, also based on in silico screening (i.a. Rathinavel et al., Bioint. Res. Appl. Chem. 11 (2021), 10161-10173; Mhatre et al., Phytomed. (2020), 153286; Chojnacka et al., J. Funct. Food 73 (2020), 104146; Ghosh et al., J. Biom. St. Dyn. (2020), 1-13-doi.org-10.1080-07391102.2020; Islam et al., Phytother. Res. 3 (2020), 2471-2492; WO 2020/037095 A1). The advantage of such compounds is that they are "generally regarded as safe" ("GRAS"; e.g. Sect. 201(s) and 409 of the U.S. Fed. Food, Drug and Cosmetic Act) and can therefore be administered without preclinical studies (also e.g. as food supplements) and might instantly be used to treat COVID-19 patients and prevent them from severe illness.

Wahedi et al. (J. Biomol. Struct. Dyn. 39 (2021): 3225-3234) suggest mainly resveratrol and i.a. also 3',4',2,4 tetrahydroxy (-trans-) stilbene as a drug candidate against COVID-19. Zhang et al. (Cell Discovery 6 (2020), 80) report that heparan sulfate assists SARS-CoV-2 in cell entry and can be targeted by approved drugs in vitro with mitoxantrone (as potent heparan sulfate inhibitor) und sunitinib and BNTX as possible candidates for such an approach. It was further reported that piceatannol binds to heparin. CN 111 228 343 A appears to disclose that an extract i.a. comprising piceatannol can be used to address infections with "2019-nCoV". Han et al. (J. Med. Virol. 87(2015): 2054-2060) report the HIV-inhibiting activity of 3,3', 4,4', 5,5' - hexahydroxy-trans-stilbene.

However, none of these substances was yet shown to have appropriate antiviral activity against SARS-CoV-2 to provide an efficient treatment or prevention option for patients infected with SARS-CoV-2.

It is therefore an object of the present invention to provide realistic and effective repurposing alternatives for the prevention or treatment of COVID-19. These alternatives should effectively prevent or hinder SARS-CoV-2 from becoming pathogenic for human patients. Accordingly, the substances should be able to inhibit SARS-CoV-2 and/or to prevent or hinder SARS-CoV-2 from entering human cells at least to a certain extent to provide significant advantage for the patient to reduce the risk of developing COVID-19 or to reduce the risk of becoming severely affected by COVID-19. Another object is that these substances are easily accessible, well tolerated and can successfully applied to humans without the need of invasive methods, preferably by inhalation, aerosol delivery, etc..

Therefore, disclosed is a compound with the general formula I 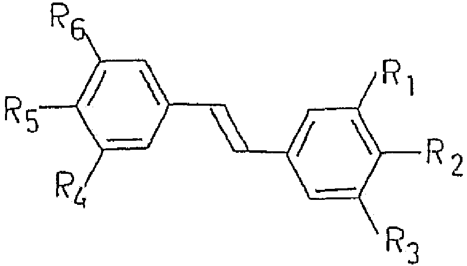 wherein R₁ to R₆ are identical or not and are H, OH-, or OR₇, wherein R₇ is a C₁ to C₃ alkyl group or a C₁ to C₄ acyl group, with the proviso that at least four, preferably at least five, especially at least six, of R₁ to R₆ are different than H, for use in the treatment and prevention of COVID-19 in a human subject, especially for inhibiting SARS-CoV-2.

The compounds were identified in the course of the present invention to exhibit realistic and effective inhibitory activity towards SARS-CoV-2. The compounds can therefore effectively prevent or hinder SARS-CoV-2 from becoming pathogenic for human patients. Accordingly, the compounds are able to inhibit SARS-CoV-2 and/or to prevent or hinder SARS-CoV-2 from entering human cells at least to a certain extent to provide significant advantage for the patient to reduce the risk of developing COVID-19 or to reduce the risk of becoming severely affected by COVID-19. The compounds are easily accessible, well tolerated and can be successfully applied to humans without the need of invasive methods. Moreover, the compounds are regarded as "GRAS", i.e. they are "generally recognised as safe (i.a. under sections 201 (s) and 409 of the US Federal Food, Drug, and Cosmetic Act.

Preferably, the compound is selected from the group 3,3',5,5'-tetramethoxystilbene, 3,4,4',5-tetramethoxystilbene, 3,3',4,5'-tetramethoxystilbene, 3,3',4,5,5'-pentamethoxystilbene, 3,3',5,5'-tetrahydroxystilbene, 3,4,4',5-tetrahydroxystilbene, 3,3',4,5'-tetrahydroxystilbene, 3,3',4,4',5-pentahydroxystilbene, 3,3',4,5,5'-pentahydroxystilbene, 3,4,4',5,5'-pentahydroxystilbene, 3,3',4',5,5'-pentahydroxystilbene, 3,3',4,4',5,5'-hexahydroxystilbene, preferably 3,3',4,5,5'-pentamethoxystilbene, 3,3',4,4',5-pentahydroxystilbene, 3,3',4,5,5'-pentahydroxystilbene, 3,4,4',5,5'-pentahydroxystilbene, 3,3',4',5,5'-pentahydroxystilbene, 3,3',4,4',5,5'-hexahydroxystilbene, especially 3,3',4,4',5,5'-hexahydroxystilbene.

The invention is defined by the claims. The compound for use according to the present invention is 3,3',4,4',5,5' -Hexahydroxy-trans-stilbene. Any subject-matter falling outside the scope of the claims, particularly subject-matter relating to stilbene derivatives other than 3,3',4,4',5,5' -Hexahydroxy-trans-stilbene, is provided for information purposes only. The compound according to the present invention has surprisingly turned out to be significantly more active in inhibiting SARS-CoV-2 than comparable polyphenolic substances suggested as being virus-inhibiting. Although also (-) epigallocatechin gallate or (-)-gallocatechin gallate show significant inhibitory effect concerning SARS-CoV-2 compared to other polyphenols, especially other compounds from green tea and black tea, the compound according to the present invention has significantly enhanced inhibitory effect over these substances.

The compound according to the present invention is preferably inhibiting replication of SARS-CoV-2 and/or for preventing the cytopathic effect of an active virus replication of SARS-CoV-2 and/or preventing viral infections with SARS-CoV-2 through airborne channels.

In the course of the present invention, the in vitro antiviral effects of nine polyphenolic plant ingredients and a synthetic polyphenolic compound, 3,3',4,4',5,5'-hexahydroxy-trans-stilbene, which was synthesized as an analogue of resveratrol, an ingredient of wine, were tested. Nine natural compounds, gallic acid, (-)-catechin, (-)-catechin gallate, (-) epigallocatechin gallate, (-)-epicatechin, (-)-epicatechin gallate, (-)-epigallocatechin, (-)-gallocatechin gallate, ellagic acid, which have been suggested in the prior art as potential antiviral compounds, and a hexahydroxy-trans-stilbene (as a representative example of synthetic resveratrol (trans-3,5,4'-trihydroxystilbene) analoga) were studied regarding their ability to inhibit virus replication in vitro.

Stilbene derivatives according to the present invention were previously disclosed e.g. in WO 02/50007 A2, WO 02/057219 A1, WO 2005/016860 A1 and WO 2007/002973 A2.

These (poly-) hydroxylated phenols were described to have increased anti-cancer and anti-inflammatory effects in comparison to resveratrol due to the increased number of OH-groups in para position on polyhydroxylated stilbenes. Hexahydroxystilbene showed most effective anti-inflammatory and anti-cancer activity in vitro and anticancer effects in animal studies and was shown to inhibit HIV infection in vitro at a very early stage. These substances were also disclosed as regulators of T cells, neutrophils, macrophages and corresponding cytokines.

In CN 1736986 A, stilbene derivates were suggested as antiviral substances for SARS-CoV-1 wherein the compounds disclosed to have antiviral activity were pyridine-group containing compounds and 2,2' -OH or CH₃O- substituted compounds as well as 3, 3' methyl-butenyl substituted compounds. Moreover, the nature of the compounds with actual anti-SARS activity were not disclosed.

All tested compounds are excellent free radical scavengers, exhibiting a broad range of biochemical effects, such as inhibiting key enzymes of DNA synthesis or inflammation. In addition, some of them were shown to inhibit different virus infections through unspecific inhibition of virus entry or replication. The compounds were selected due to their chemical structure and availability in natural sources such as tea or fruits.

All compounds were investigated regarding their inhibitory effects on virus infection using in vitro cell culture models.

The receptor for docking of SARS-CoV-2 on to the cell is the ACE 2 receptor. Binding studies were performed for three compounds, which showed most effective anti-viral effects.

In contrast to other suggestions in the prior art in which several polyphenolic substances from green and black tea were described to have potential anti-SARS-CoV-2 properties, it turned out with the present invention that polyphenolic substances highly vary in their effect against SARS-CoV-2 and that only a few of these substances are effective.

These experiments performed in the course of the present invention showed that the synthetic resveratrol analoga according to the present disclosure, preferably the tetra-, penta- and hexa-hydroxy-forms thereof, especially hexahydroxystilbene, exhibit inhibiting activity against SARS-CoV-2 which significantly outperforms other natural polyphenolic compounds, even the few natural polyphenolic compounds which also turned out to have a real-world antiviral activity against SARS-CoV-2 (in contrast to the optimistic prediction based on analogies with other viruses or computer predictions).

The compound of the present invention is in principle known as pharmaceutical substance. Accordingly, the formulations already known and proven to be effective for delivery of this compound to the human subject are also applicable for the present invention. The route of administration can be divided into enteral or parenteral, such as oral, sublingual, intramuscular, subcutaneous, nasal, oral mucosa, skin, peritoneum or rectum, etc., preferably oral, sublingual or nasal, especially as an inhalable and/or aerosol composition. The compound of the present invention or the pharmaceutical composition containing it can be administered in a unit dosage form. The dosage form for administration can be a liquid dosage form or a solid dosage form. For example, the liquid dosage form can be a true solution type, colloid type, microparticle dosage form, emulsion type, or suspension type. Examples of dosage forms include tablets; caplets; capsules, such as hard gelatin capsules and soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); sublingual films or tablets; lollipops; gels; liquid dosage forms suitable for oral or mucosal administration to a patient, including suspensions (e.g., aqueous or non-aqueous liquid suspensions, oil-in-water emulsions, or water-in-oil liquid emulsions), solutions, and elixirs. The compounds of the present invention can be made into ordinary preparations, and can also be slow-release agents, controlled-release agents, targeted preparations, and various particulate drug delivery systems.

Therefore, the present invention relates to a pharmaceutical preparation comprising the compound according to the present invention with a pharmaceutically acceptable excipient for use according to the appended claims. A pharmaceutically acceptable excipient may be any excipient known to be suitable and used for the compound according to the present invention, especially a carrier or diluent.

In order to make a unit dosage form, various carriers known in the art can be widely used.

Examples of carriers or diluents are, for example, absorbents, such as starch, dextrin, calcium sulfate, lactose, mannitol, sucrose, sodium chloride, glucose, urea, kaolin, microcrystalline cellulose, aluminium silicate, etc.; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone, etc.; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose, etc.; disintegration inhibitors, such as sucrose, glyceryl tristearate, cocoa butter, hydrogenated oil, etc.; absorption enhancers, such as quaternary ammonium salts, sodium lauryl sulfate, etc.; lubricants, such as talc, silicon dioxide, corn starch, stearic acid, boric acid, liquid paraffin, polyethylene glycol, etc.. Other carriers such as polyacrylic resins, liposomes, water-soluble carriers such as PEG4000 and PEG6000, PVP and so on. The tablets can also be further made into coated tablets, such as sugar-coated tablets, film-coated tablets, enteric-coated tablets, or double-layered and multi-layered tablets. For example, in order to make the administration unit into a pill, various carriers known in the art can be widely used. Examples of carriers are, for example, diluents and absorbents, such as glucose, lactose, starch, cocoa butter, hydrogenated vegetable oil, polyvinylpyrrolidone, kaolin, talc, etc.; binders, such as acacia, tragacanth, gelatin, Ethanol, honey, liquid sugar, rice paste or batter, etc.; disintegrants, such as agar powder, dried starch, alginate, sodium lauryl sulfonate, methyl cellulose, ethyl cellulose, etc.. For example, in order to make the dosing unit into a capsule, the compound of the present invention as an active ingredient is mixed with the aforementioned various carriers, and the resulting mixture is placed in a hard gelatin capsule or a soft capsule. The active ingredient of the compound of the present invention can also be made into microcapsules, suspended in an aqueous medium to form a suspension, or filled into hard capsules or made into injections, inhalations or aerosols, for application. For example, the compound of the present invention is prepared into an injection, an inhalation or aerosol preparation, such as a solution, a suspension solution, an emulsion, a lyophilized powder, and this preparation may be aqueous or non-aqueous.

The pharmaceutical preparation of the present invention may contain one and/or more pharmacodynamically acceptable excipients, such as carriers, diluents, binders, lubricants, preservatives, surfactants or dispersants. For example, the diluent can be selected from water, ethanol, polyethylene glycol, 1,3-propanediol, ethoxylated isostearyl alcohol, polyoxylated isostearyl alcohol, polyoxyethylene sorbitol fatty acid ester, etc.. In addition, in order to prepare an isotonic inhalation solution/suspension, aerosol, injection, an appropriate amount of sodium chloride, glucose or glycerin can be added to the injection preparation. In addition, conventional solubilizers, buffers, pH adjusters, etc. can also be added. These auxiliary materials are commonly used in this field. In addition, if necessary, colouring agents, preservatives, flavours, sweeteners, or other materials can also be added to the pharmaceutical preparation.

In order to achieve the purpose of medication and enhance the therapeutic effect, the drug or pharmaceutical composition of the present invention can be administered by any known administration method. The dosage of the extract or compound or pharmaceutical composition of the present invention depends on many factors, such as the nature and severity of the disease to be prevented or treated, the gender, age, weight, personality and individual response of the patient or animal, the route of administration, the number of administrations and the purpose of treatment, so the therapeutic dose of the present invention can have a wide range of changes. Generally speaking, the dosage of the pharmacodynamic ingredient of the present invention is well known to those skilled in the art. According to the actual amount of the drug contained in the final formulation of the compound composition of the present invention, appropriate adjustments can be made to meet the requirements of the therapeutically effective dose, and the dosage of the compound of the present invention can be completed, most preferably 0.1-20 mg/Kg body weight.

The above-mentioned dosage can be administered in a single dosage form or divided into several, for example, two, three or four dosage forms, which is limited by the clinical experience of the administering doctor and the dosage regimen including the use of other treatment means.

According to a preferred embodiment, the polyphenolic compound according to the present invention is formulated for delivery into the upper respiratory system. Exemplary formulations include nasal, bronchial, oral, and pulmonary formulations. However, the compound according to the present invention may also be formulated for topical administration including a liquid, gel, wax, or paste. It is specifically preferred to formulate the present composition as an aerosol. The aerosol can be a liquid or powdered aerosol. In some embodiments, the composition contains one or more pharmaceutically acceptable excipients such as glycerol. The composition can contain 0.01%-20% w/v of the effective ingredient according to the present invention and 10% to 20% glycerol.

Pharmaceutical compositions and unit dosage forms of the disclosure typically also include one or more pharmaceutically acceptable excipients, especially carriers or diluents. Advantages provided by specific compounds of the disclosure, such as increased solubility and/or enhanced flow, purity, or stability (e.g., hygroscopicity) characteristics can make them better suited for pharmaceutical formulation and/or administration to patients than the prior art.

Suitable excipients are well known to those skilled in the art of pharmacy or pharmaceutics. Whether a particular excipient is suitable for incorporation into a pharmaceutical composition or dosage form depends on a variety of factors well known in the art including the way in which the dosage form will be administered to a patient. For example, oral dosage forms such as tablets or capsules may contain excipients not suited for use in parenteral dosage forms. The suitability of a particular excipient may also depend on the specific active ingredients in the dosage form. For example, the decomposition of some active ingredients can be accelerated by some excipients such as lactose, or when exposed to water. Active ingredients that include primary or secondary amines are particularly susceptible to such accelerated decomposition.

Specifically preferred pharmaceutically acceptable excipients are antioxidants (reduction agents). Antioxidants commonly used in pharmaceutical compositions include citric acid and its salts (E330-E333), tartaric acid and its salts (E334-E337), phosphoric acid and its salts (E338-E343) and ethylenediaminetetraacetic acid (EDTA) and its salts (calcium disodium EDTA, E385), vitamins C, E, etc..

The disclosure further encompasses pharmaceutical compositions and dosage forms that include one or more compounds that reduce the rate by which an active ingredient will decompose. Examples of such compounds are stabilizers, such as antioxidants such as ascorbic acid, pH buffers, or salt buffers. In addition, pharmaceutical compositions or dosage forms of the disclosure may contain one or more solubility modulators, such as sodium chloride, sodium sulfate, sodium or potassium phosphate or organic acids. A specific solubility modulator is tartaric acid.

Like the amounts and types of excipients, the amounts and specific type of compounds according to the present invention in a dosage form may depend on factors such as the route by which it is to be administered to patients. Typical dosage forms of the compound of the present invention contain the effective ingredient according to the present invention in an amount of from about 100 pg to about 10 g, preferably in an amount of from about 1 mg to about 1 g, more preferably in an amount of from 10 mg to 500 mg, even more preferably in an amount of from about 20 mg to about 300 mg. Preferred dosage forms contain the compound of the present invention in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.

Preferably, the pharmaceutical compositions may also include a carrier, for example a sugar alcohol such as but not limited to glycerol, mannitol, sorbitol, xylitol, and erythritol. In a specific embodiment, the sugar alcohol is glycerol.

Typical topical dosage forms include liquids, creams, lotions, ointments, gels waxes, pastes, sprays, aerosols, solutions, emulsions, and other forms know to one of skill in the art. In a preferred embodiment, the present compounds are delivered to oral, nasal, or bronchial tissue in a suitable topical dosage form.

For non-sprayable topical dosage forms, viscous to semisolid or solid forms including a carrier or one or more excipients compatible with topical application and having a dynamic viscosity preferably greater than water are typically employed. Suitable formulations include solutions, suspensions, emulsions, creams, ointments, powders, gels, waxes, pastes, liniments, salves, and the like, which are, if desired, sterilized or mixed with auxiliary agents (e.g., preservatives, stabilizers, wetting agents, buffers, or salts) for influencing various properties, such as, for example, osmotic pressure.

Nasal spray drug products contain therapeutically active ingredients dissolved or suspended in solutions or mixtures of excipients in non-pressurized dispensers that deliver a spray containing a metered dose of the active ingredient. The dose can be metered by the spray pump or could have been pre-metered during manufacture. A nasal spray unit can be designed for unit dosing or can discharge up to several hundred metered sprays of formulation containing the drug substance. Nasal sprays are applied to the nasal cavity for local and/or systemic effects.

According to another preferred embodiment, the compound according to the present invention is provided as inhalation solution and suspension drug products. Such products are typically aqueous-based formulations that contain therapeutically active ingredients and can also contain additional excipients. Aqueous-based oral inhalation solutions and suspension must be sterile. Inhalation solutions and suspensions are intended for delivery to the lungs by oral inhalation for local and/or systemic effects and are to be used with a specified nebulizer. An inhalation spray drug product consists of the formulation and the container closure system. The formulations are typically aqueous based and must be sterile. Inhalation sprays are intended for delivery to the lungs by oral inhalation for local and/or systemic effects. Other suitable topical dosage forms include sprayable aerosol preparations wherein the active ingredient, preferably in combination with a solid or liquid inert carrier, is packaged in a mixture with a pressurized volatile (e.g., a gaseous propellant, such as freon), or in a squeeze bottle. Examples of sprayable aerosol preparations include metered dose inhalers, dry powder inhalers, and nebulizers. Moisturizers or humectants can also be added to pharmaceutical compositions and dosage forms if desired.

It may also be advantageous to provide the pharmaceutical compositions according to the present invention in transdermal and mucosal dosage forms, such as ophthalmic solutions, patches, sprays, aerosols, creams, lotions, suppositories, ointments, gels, solutions, emulsions, suspensions, or other forms known to one of skill in the art. Dosage forms suitable for treating mucosal tissues within the oral cavity can be formulated as mouthwashes, as oral gels, or as buccal patches. Additional transdermal dosage forms include reservoir type or matrix type patches, which can be applied to the skin and worn for a specific period of time to permit the penetration of a desired amount of active ingredient. Suitable excipients (e.g., carriers and diluents) and other materials that can be used to provide transdermal and mucosal dosage forms are well known to those skilled in the pharmaceutical field, and depend on the particular tissue or organ to which a given pharmaceutical composition or dosage form will be applied. With that fact in mind, typical excipients include water, acetone, ethanol, ethylene glycol, propylene glycol, butane-1,3-diol, isopropyl myristate, isopropyl palmitate, mineral oil, and mixtures thereof, to form dosage forms that are non-toxic and pharmaceutically acceptable. Depending on the specific tissue to be treated, additional components may be used prior to, in conjunction with, or subsequent to treatment with the compounds according to the present invention. For example, penetration enhancers can be used to assist in delivering the active ingredients to or across the tissue. Suitable penetration enhancers include acetone; various alcohols such as ethanol, oleyl, a tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as TWEEN 80 (polysorbate 80) and SPAN 60 (sorbitan monostearate).

The pH of a pharmaceutical composition or dosage form, or of the tissue to which the pharmaceutical composition or dosage form is applied, may also be adjusted to improve delivery of the active ingredient(s). Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of the active ingredient(s) so as to improve delivery. In this regard, stearates can serve as a lipid vehicle for the formulation, as an emulsifying agent or surfactant, and as a delivery-enhancing or penetrationenhancing agent.

The compound of the present invention can also be formulated as extended or delayed release formulations. Extended and delayed release formulations for various active ingredients are known in the art, for example by encapsulation.

The compound of the present invention is present in the pharmaceutical composition in about 0.001% to about 50% w/v, typically from about 0.01% to about 0.1% w/v, more typically about 1 % to about 20% w/v. In a preferred embodiment, the compound of the present invention is present in about 0.01% to about 20% w/v. The pharmaceutically acceptable excipient and eventually other compounds or agents present in the pharmaceutical composition then add up to the 100%.

The compound and compositions of the present invention are useful for the treatment of one of more symptoms of viral infection with SARS-CoV-2. Preferably, the pharmaceutical compositions according to the present invention are formulated for nasal or oral application, such as drops, applicators, or sprays. One embodiment provides the compositions according to the present invention for prophylactically or therapeutically treating patients which are infected by or are at risk of being infected by SARS-CoV-2, especially for use to prevent viral infections through airborne channels.

The present invention is further illustrated by the following examples and the drawing figures, yet without being restricted thereto.

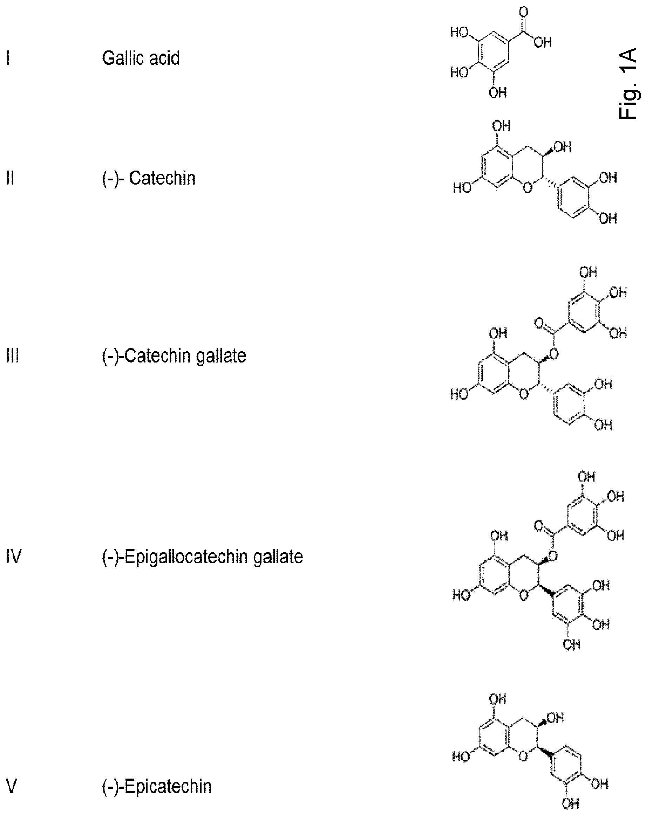

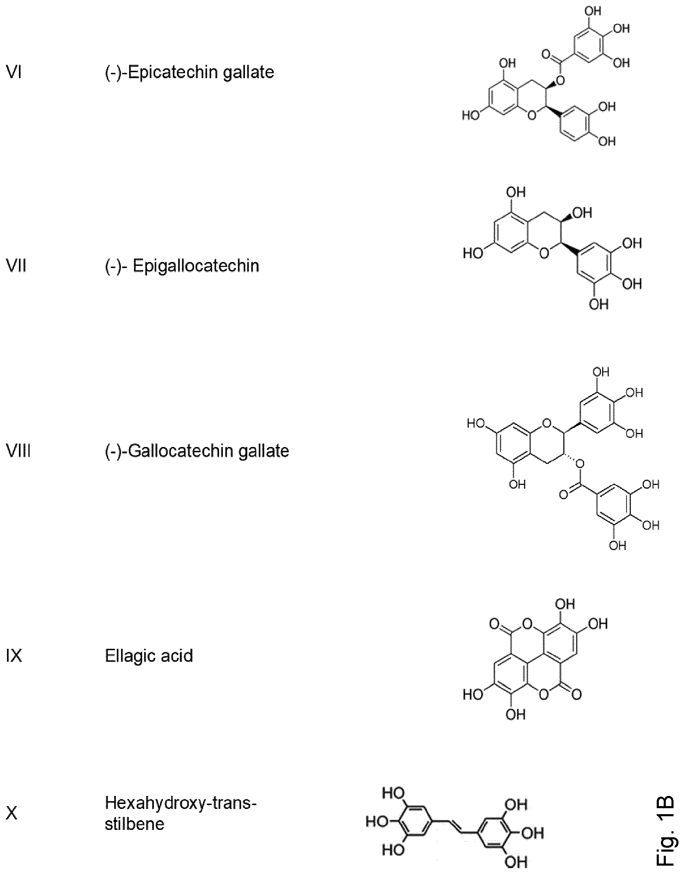

Fig. 1 shows the compounds tested and their chemical structure; compound I: gallic acid, compound II: (-)-catechin, compound III: (-)-catechin gallate, compound IV: (-) epigallocatechin gallate, compound V: (-)-epicatechin, compound VI: (-)-epicatechin gallate, compound VII: (-)-epigallocatechin, compound VIII: (-)-gallocatechin gallate, compound IX: ellagic acid, and compound X: hexahydroxy-trans-stilbene;
Fig. 2 shows the effect of preincubations with indicated substances on TCID50 of SARS-CoV-2
Fig. 3, 4 and 5 show the performance of compounds IV, VIII and X in a Vero cell culture assay with SARS-CoV-2 infected cells, wherein cells were incubated then infected and virus load was determined after 48h incubation with different concentrations of the compounds; RT PCR assay in the supernatant was used to quantify virus infection;

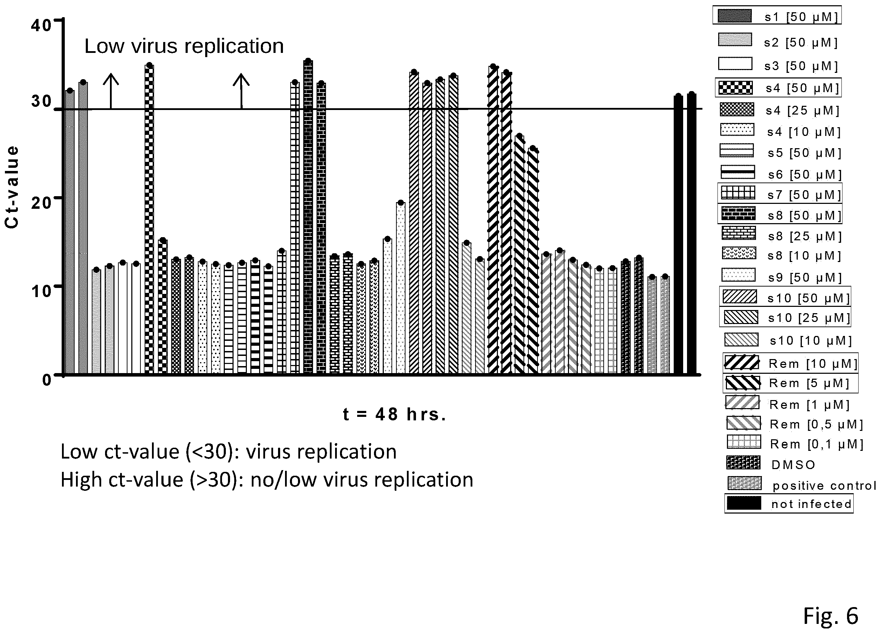

Figs. 6 and 7 show RT PCR assays in the supernatant to quantify virus infection of compounds I to X ("s1" to "s10") and remdesivir;
Fig. 8 shows two runs of virus inhibition;
Fig. 9 shows the structure of resveratrol, piceatannol and hexahydroxystilbene (compound X);
Fig. 10 shows the viral copy numbers at different concentrations of compounds (dots: resveratrol, squares: piceatannol, triangles up: hexahydroxystilbene (compound X); triangles down: positive control);
Fig. 11 shows the viral copy numbers at different concentrations of compounds wherein a RT PCR assay in the supernatant was used to quantify virus infection; the column at the right side is the positive control: (A) resveratrol, (B) piceatannol, (C) hexahydroxystilbene (compound X);
Figs 12 to 14 show the performance of hexahydroxystilbene (compound X), resveratrol, and piceatannol in a Vero cell culture assay with SARS-CoV-2 infected cells, wherein cells were incubated then infected and virus load was determined after 48h incubation with different concentrations of the compounds.

### Examples:

In the present examples, the SARS-CoV-2 inhibiting effects of compounds I to X were investigated, including TCID50 of SARS-CoV-2 and a Vero cell culture assay with SARS-CoV-2 infected cells (only compound X is according to the invention; the other tested compounds are reference compounds).

### Materials and Methods

Cells were infected and incubated with different concentrations of all compounds.

### Median tissue culture infective dose (TCID₅₀) assay

Vero cells were plated in growth medium (DMEM containing 10% FCS, 100µM non-essential amino acids, 1mM sodium pyruvate and penicillin/streptomycin) into a 96-well plate at a density of 1.5-2 × 10⁴/well on the evening before the experiment. On the next day in the morning, growth medium was removed and 95% confluent Vero cells were preincubated for 45 minutes with either DMSO or substance I, IV, VII, VIII and X at a dose of 50 µM and infected with 100 µL of serial dilutions of a SARS-CoV-2 virus stock. Preincubation and Virus infection was carried out in medium containing only 2%FCS instead of 10% FCS. After 5 to 7 days all wells were checked for cell viability and the TCID₅₀ dose in the presence or absence of indicated substances was calculated using the Reed and Munch method (Reed et al., Am. J. Epidemiol. 27 (1938), 493- 497). Wells with viable and infected cells were identified using an inverse light microscope showing either a nicely visible and intact monolayer of Vero cells (viable), or wells with a massive CPE (cytopathic effect), characterized by a large amount of dead cells and the absence of a monolayer (infected) .

After 5 to 7 days all wells were checked for cytopathic effects (CPE) and the TCID₅₀ dose in the presence or absence of indicated substances was calculated using the Reed et al. method.

### Cell culture

African green monkey kidney epithelial cells VeroE6 (obtained from biomedica) cells were maintained in Gibco's Minimum Essential Medium supplemented with Earle's Salts and L-Glutamine (Gibco 11095-080, 500 mL) with 5 % FCS and 1 % PenStrep, in the following referred to as MEM 5 %. Incubation at 37 °C, 5 % CO₂ if not stated otherwise.
Virus: Stock preparation and titre determination
Viral SARS-CoV-2 strain: Human 2019-nCoV Isolate

Product Description Ref-SKU: 026V-03883 Infectious cell culture supernatant of human 2019-nCoV Product Risk Group: RG3 ICTV Taxonomy:
ssRNA(+)/Nidovirales/Coronaviridae/Coronavirinae/Betacoronavirus Virus name: Human 2019-nCoV ex China Strain: BavPat1/2020 Isolate: Germany ex China

Based on this stock solution of 2.2 E+06 PFU/mL of Human 2019-nCoV Isolate, a viral working stock (VPN3) was cultured in EMEM (Gibco) + 10 % FCS. For the working stock, titre determination via TCID₅₀ and Plaque assay were made in parallel and revealed a concentration of 1,30E+06 viral RNA copies per µL stock. Aliquots were stored at -80 °C. For the infection assays, the working stock was thaw and diluted in MEM 2 % to a MOI of 0,002.

The European Commission classifies SARS-CoV-2 as a risk group 3 pathogen. Experiments with active virus and high concentration virus stocks require working under biosafety level 3 conditions (BSL-3). At the Institute of Pathology at the Medical University of Graz, all working steps with the active virus were performed under BSL-3 conditions and with an increased personal safety equipment, to avoid transmission of virus via aerosols.

### Substances

Substances were dissolved in DMSO (Sigma) to substance stock concentrations of 5 mM or 1 mM, respectively. Further 1:2 dilutions were made to gain 2,5 mM and 0,5 mM stocks from certain substances. For the final assay concentration, a 1:100 dilution of the substance stocks in MEM 2 % per well was made. Substance stocks were prepared freshly prior to every assay.

### Cytotox assay

For cytotoxicity assays VeroE6 cells were seeded in 96-well plates with 8500 to 10000 cells per well, using MEM supplemented with 2 % FCS, 24 hrs. before substance treatment. Incubation at 37 °C, 5 % CO₂.

After incubation, the cells were exposed to 1:100 dilutions of substances stocks in MEM + 2 % FCS (as described above). Triplicates per substance and concentration were tested. Directly after substance addition, 24 hrs. and 48 hrs. later, the metabolic activity was measured using a resazurin-based assay. After the addition of Resazurin (10 µM concentration) the increase of relative fluorescent units (RFU) was measured for 3 hrs. and a linear regression analysis was performed. Slopes of substance treated cells were normalized to that of untreated cells (untreated cells = cells + respective amount of medium) to calculate the relative metabolic activity.

### Infection assay

VeroE6 Cells used in infection assays were seeded with a density of 2,5 × 10⁴ to 3,0 × 10⁴ cells per well (300 µL) in a 48-well plate (Corning Costar, cell culture treated) in MEM + 2 % FCS 24 hrs. prior, if not stated otherwise.

At the infection day the seeding medium was removed and the cells were treated with medium (MEM 2 % FCS) containing 1 % DMSO + dissolved substances in certain concentrations) in a final volume of 198 µL. Subsequently, 2 µL of the diluted virus stock containing SARS-CoV-2 (calculated to a MOI 0,002) were added. The cells were infected with virus for 1 h at 5 % CO₂ and 37 °C. After the incubation step, the infection medium was removed and the cells were washed 2 times with MEM without FCS. 440 µl fresh MEM 2 % FCS, either containing substances or not were given on the cells. 10 minutes later, the supernatant for time point 0 was collected (140 µL) and frozen at -80 °C or inactivated with 560 µl AVL buffer.

The cells were incubated for additional 48 hrs. at 5 % CO₂ and 37 °C until supernatant was harvested for time point 48 and inactivated with 560 µl AVL buffer. In addition, either cells were lysed in the well for intracellular virus detection (RTX buffer from Qiagen RNeasy Kit, followed by RNA preparation based the protocol of this kit), or the plate was fixed in 4 % formalin for SARS- specific immune histochemical staining (IHC).

### RNA isolation and RT-qPCR

After inactivation of the supernatant samples containing virus with AVL buffer (Qiagen), the viral RNA was isolated using the QIamp viral RNA mini Kit (QiAmp Viral RNAMini Kit, Qiagen), following the manufacture's protocol as recommended by CDC. The RNA was eluated in 40 µL ultra-pure H₂O and stored at -80 °C.

The RT-qPCR, to detect the viral load of the samples, was performed based on the CDC recommendation using QuantiTect Multiplex RT-PCR Kit with a Rotor Gene Q cycler:
2019-nCoV_N1-F 2019-nCoV_N1 Forward Primer 5'-GAC CCC AAA ATC AGC GAA AT-3'
2019-nCoV_N1-R 2019-nCoV_N1 Reverse Primer 5'-TCT GGT TAC TGC CAG TTG AAT CTG-3'
2019-nCoV_N1-P 2019-nCoV_N1 Probe 5'-FAM-ACC CCG CAT TAC GTT TGG TGG ACC-BHQ1-3' FAM, BHQ-1

### Rotorgene

qRT-PCR analysis was performed using Rotorgene and following the manufacturer's protocol.

| 25 µL approach | |
|---|---|
| QuantiTect Mastermix | 12,5 µl |
| Primer forward | 1 µl |
| Primer reverese | 1 µl |
| Qt Probe | 0,5 µl |
| RT-Mix | 0,25 µl |
| water | 4,75 µl |
| sample | 5 µl |
| Total volume | 25 µl |

| Temperature profile for amplification | |
|---|---|
| | ∘ Hold 1: 50°C, 30 Min |
| | ∘ Hold 2: 95°C, 15 Min |
| | ∘ Cycling (2 Step Cycling): 95°C, 3 sec + 55°C, 30 sec |
| | ∘ Cycle: 45 |

### Immunohistochemistry

After fixation of the cells with 4 % formalin and washing with PBS, the cells were permeabilized using 0,1 % Triton X 100 in PBS for 10 min (200 µl per well), followed by 3 washing steps with 200 µl PBS. The endogen peroxidases were blocked with 3 % H₂O₂ in methanol for 30 min. After this, 3 washing steps with 200 µl PBS followed and the cells were incubated for 1 h with 100 µL primary antibody (SARS-CoV-2 nucleocapsid; 1:1000 dilution in antibody diluent) per well. 3 washing steps with 200 µl PBS followed and during another incubation step, the cells were treated with the secondary antibody (EnVision) for at least 30 min. (protected from light).

After washing (PBS 3x), the substrate AEC was dropped (2 drops) on the cells and incubated until viral infected cells were stained red (watch under the microscope) but not later than 3 minutes. Reaction was stopped with PBS washing (3x). Wells were kept in PBS until photo documentation.

| Reagent: | Company | Cat # |
|---|---|---|
| SARS-CoV-2 (2019-nCoV) Nucleocapsid Antibody, RabSinobiological | | 40143-R019 |
| bit MAb | | |
| REAL Antibody Diluent | Agilent Technologie, Dako | S202230-2 |
| EnVision^{™} + Dual Link System HRP | Agilent Technologie, Dako | K5007 |
| AEC Substrate-Chromogen | Agilent Technologie, Dako | K346430-2 |

### Molecular interaction assay to detect inhibition of RBD to ACE2 receptor binding

The molecular interaction assay to detect inhibition of RBD to ACE2 receptor binding was performed as described (Gattinger et al., Allergy, 76 (2021), 878-883) with following alterations: 200 ng His-tagged RBD was incubated with various doses (100, 50, 25, 12.2, and 6 mM) of substance X for three hours at RT followed by a 3-hours overlay onto plate bound ACE2 (2µg/ml). Bound RBD was then detected with a mouse monoclonal anti-His antibody followed by an HRP-labelled anti-mouse IgG₁ antibody and detected with ABTS detected. All measurements were performed in duplicates with a variation of <5%.

### Results

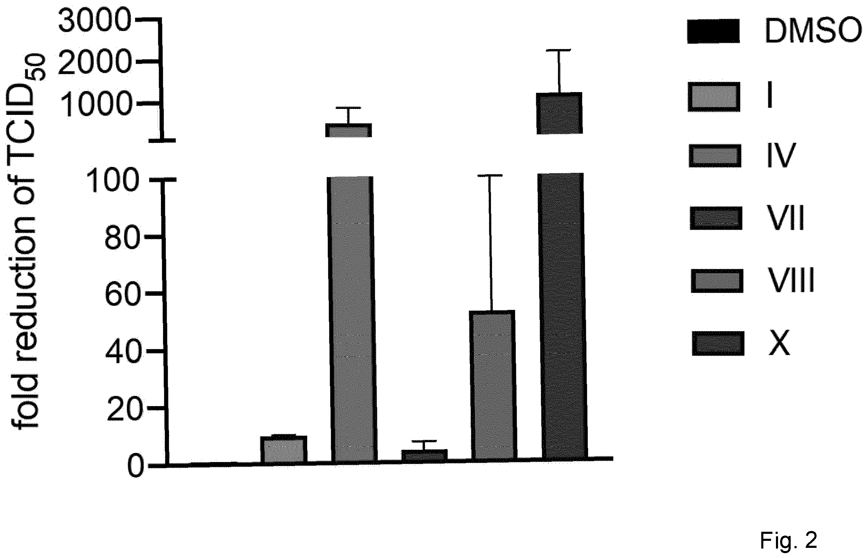

In a first set of experiments, the TCID₅₀ of SARS-CoV-2 was analysed using Vero cells after preincubation with DMSO or indicated substances (I-X) dissolved in DMSO was determined. As expected DMSO reduced the TCID₅₀ of SARS-CoV-2 by 1 Log. Cells were seeded and then infected for 1 hour, then compounds were added in different concentration for 48 hours. All substances showed cell protective effects and reduced the TCID₅₀ by at least 1 Log scale. TCID₅₀ was significantly lowered by 50 µM of substances IV (99,7- and 802-fold reduction), VIII (99,7- and 5,14-fold reduction) and X (99,7 and 2107 fold reduction) showing inhibition of virus replication (Table 1 and Figure 2).

**Table 1:**

| Experiment 1: TCID₅₀ assay with 2 doses/substance [TCID50/mL] | | | Experiment 2: repeat of TCID₅₀ assay with a dose of 50uM [TCID₅₀/mL] | | Experiment 3: repeat of TCID₅₀ assay with a dose of 50uM [TCID₅₀/mL] | |
|---|---|---|---|---|---|---|
| Substance | 10 µM | 100 µM | 50 µM | Fold reduction | 50 µM | Fold reduction |
| I | 3,16 × 10⁵ | 3,56 × 10⁵ | 3,16 × 10⁴ | 9,97 | 4,44 × 10⁵ | 9,27 |
| IV | 3,36 × 10⁴ | *cytotoxic dose* | 3,16 × 10³ | 99,7 | 5,12 × 10³ | 802,3 |
| VII | 2,15 × 10⁴ | 3,16 × 10³ | 2,89 × 10⁵ | 1,09 | 3,80 × 10⁵ | 7,09 |
| VIII | 2,15 × 10⁵ | 4,64 × 10² | 3,16 × 10³ | 99,7 | 8 × 10⁵ | 5,14 |
| X | 5,88 × 10⁴ | 4,64 × 10² | 3,16 × 10³ | 99,7 | 1,95 × 10³ | 2107,2 |
| Untreated ctrl. | n.a. | 1 × 10⁶ | n.a. | - | n.a. | - |
| DMSO ctrl | n.a. | 1 × 10⁵ | 3,15 × 10⁵ | - | 4,11 × 10⁶ | - |

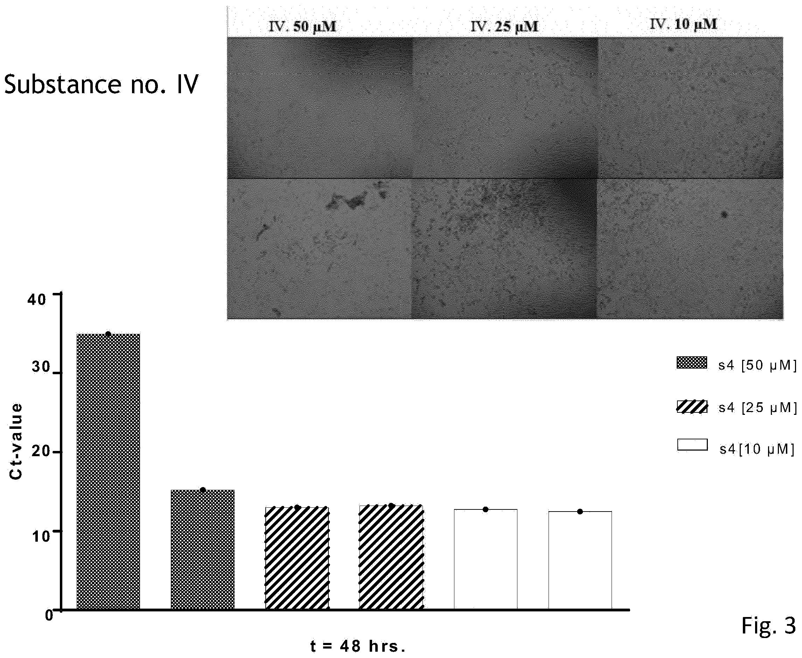

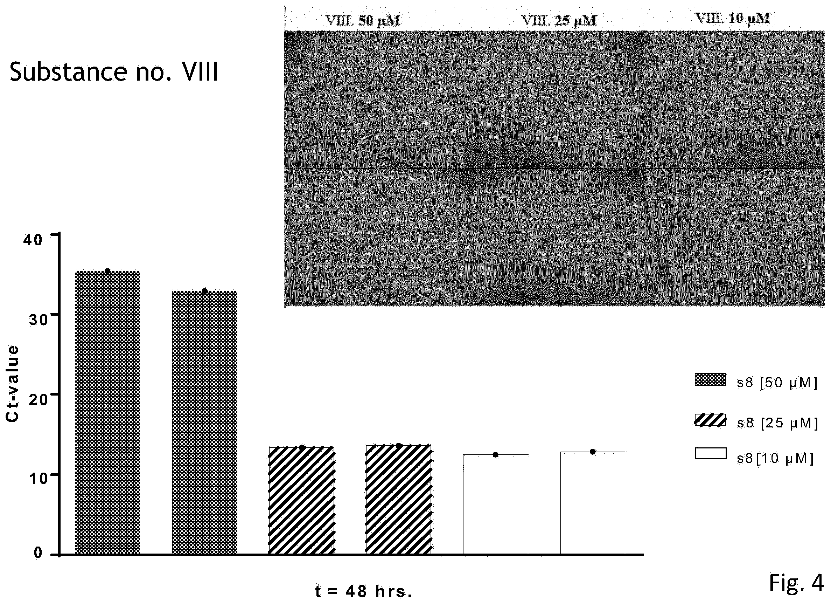

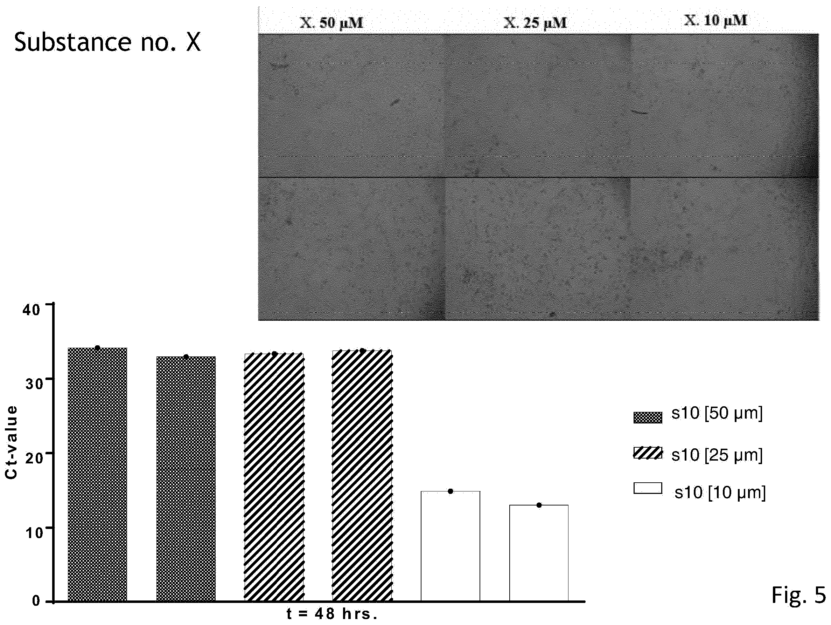

The in vitro inhibition of virus proliferation was repeated in another laboratory:
Vero cells were seeded in 48 well plates as described in the methods section and then cells were infected and incubated with different concentrations of all 10 compounds for 48 hours. Compounds IV, and VIII and X showed inhibition of virus proliferation which is in line with the results seen in the first set of experiments (TCID₅₀ assay). Then qPCR of SARS-CoV-2 was performed in the supernatant after RNA isolation. Compound IV caused an increase of ct values above 30 at concentration of 50 µM in one of two duplicates. An increase of ct values greater 30 were also seen after incubation with 50 µM substance VIII. Compound X caused increased ct values (above 30) after treatment with 25 and 50 µM for 48 hours showing inhibition of virus proliferation (see also: Figs. 3, 4 and 5).

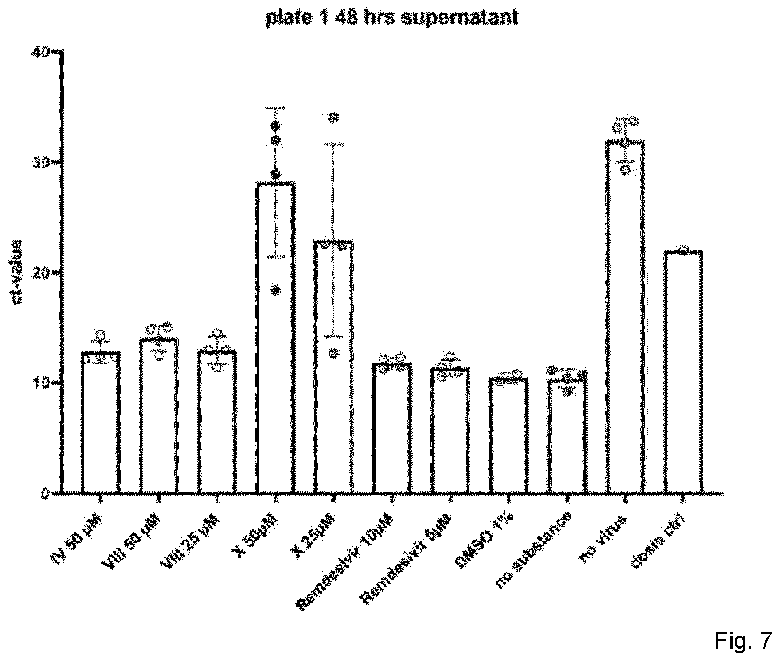

In order to determine whether inhibition of virus proliferation was due to alterations of virus uptake into the host cells, the cells were further incubated with compounds IV, VIII and X (the compounds which proved effective in previous experiments) for one hour together with cell infection and then the compounds were washed out. Cell supernatant was then tested by qPCR as described above. Interestingly, compounds IV and VIII did not have any effects on the virus proliferation in comparison with untreated controls, while compound X could inhibit virus proliferation in a concentration dependent manner after short term incubation for one hour. Results are shown in figure 7; ct values significantly increased after incubation with 25 or 50 µM for one hour.

This result shows that virus uptake into the host cell could be inhibited by compound X. It was therefore investigated whether compound X is capable to block ACE2 receptor, which was described to act as entry of the virus into the cell.

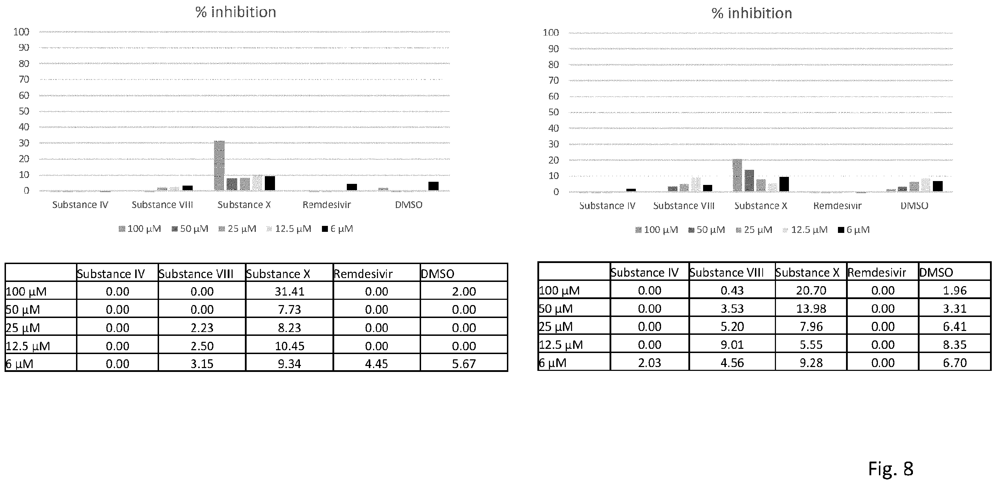

The effects of compounds IV, VIII and X on the ACE2 receptor were then tested. Compounds IV and VIII did not show any inhibitory effects on the binding of ACE 2 receptor in this test. Compound X could inhibit binding to ACE2 in a concentration dependent manner. At 100 µM it inhibited ACE2 binding between 21 and 31 percent (see Fig. 8). This shows that compound X can inhibit virus uptake into the cell at a very early stage, which at least in part can be attributed to the binding of the compound to the ACE2 receptor.
Comparative example with respect to trihydroxystilbene (resveratrol), tetrahydroxystilbene (piceatannol) and hexahydroxystilbene (compound X according to the present invention)

Comparative tests with respect to resveratrol, piceatannol and hexahydroxystilbene, compound X according to the present invention, was carried out as disclosed above. Vero E6 cells were uses as cellular system. Cells were infected with the virus for 1 h, then the test compound was added. Then washing and incubation with the test compound for 48 hrs was performed. Results/readout were obtained by qPCR of the supernatant and IHC.

### Results

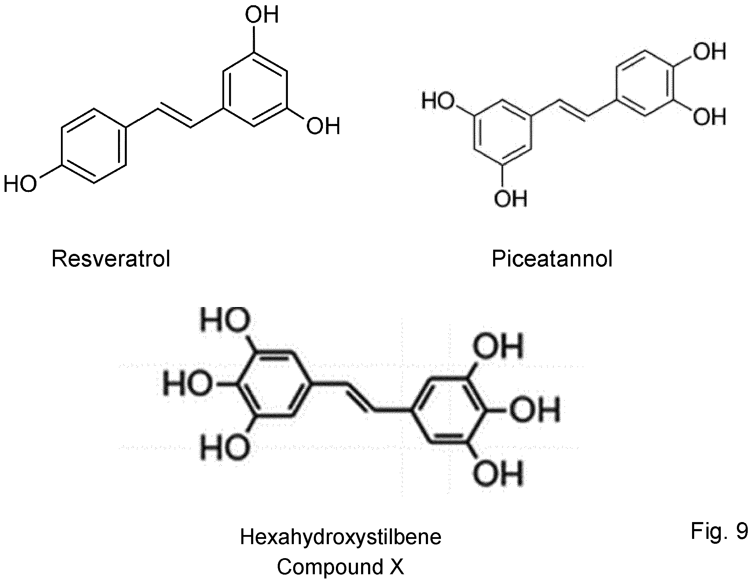

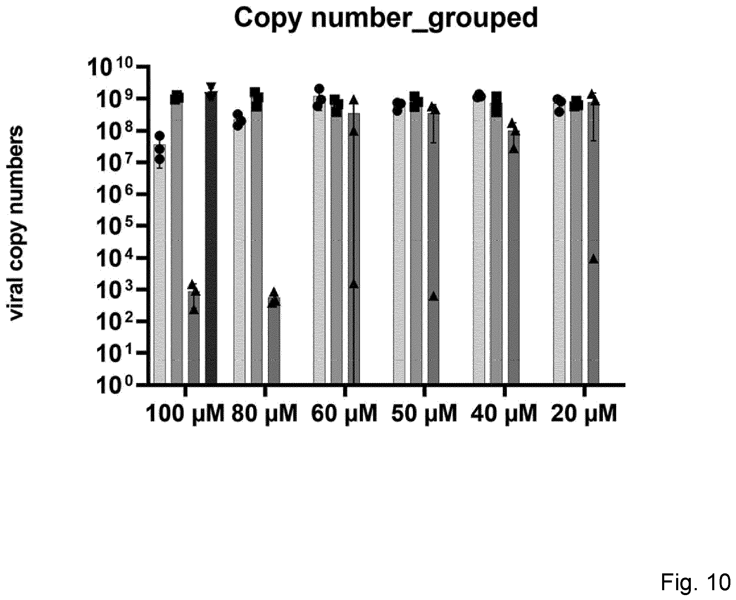

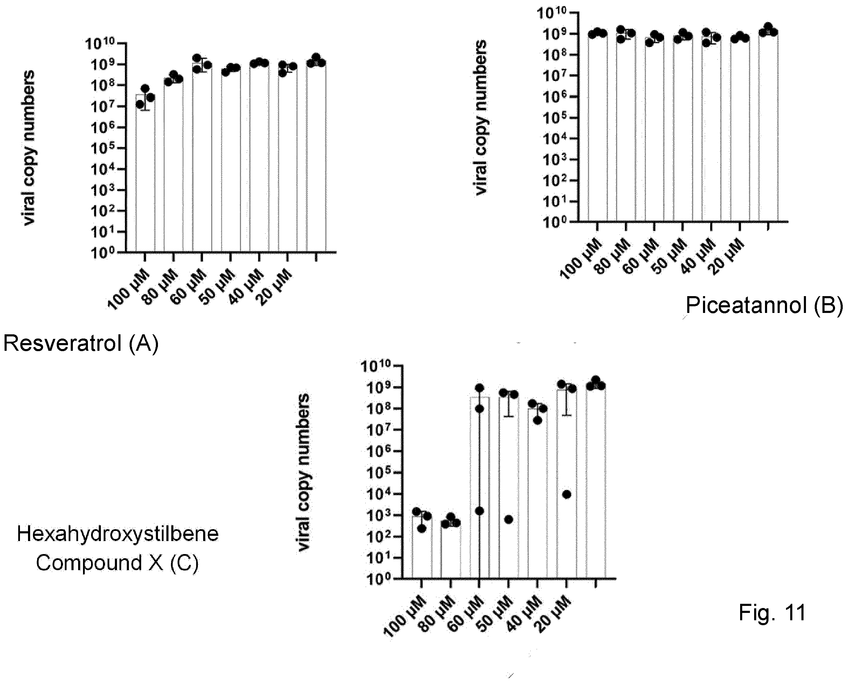

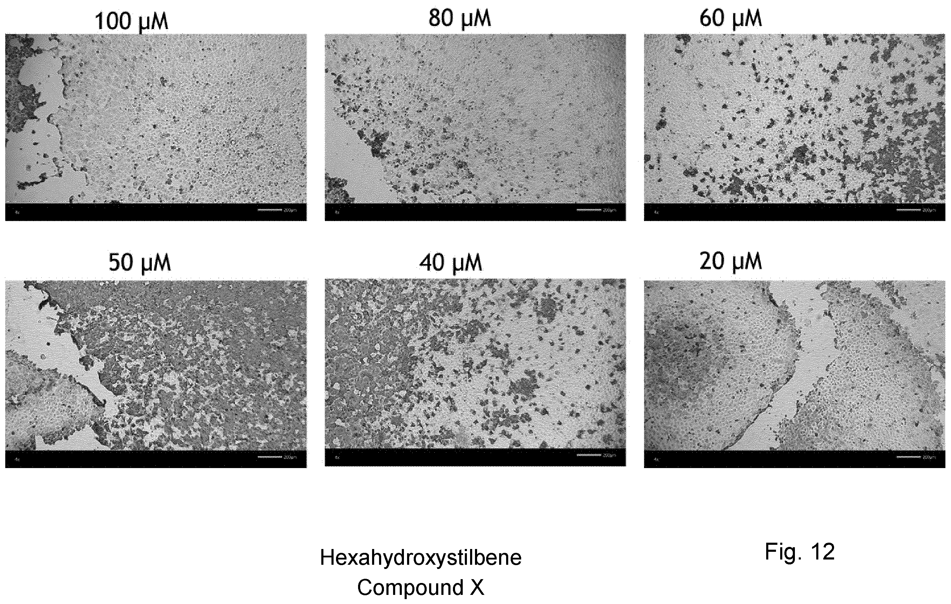

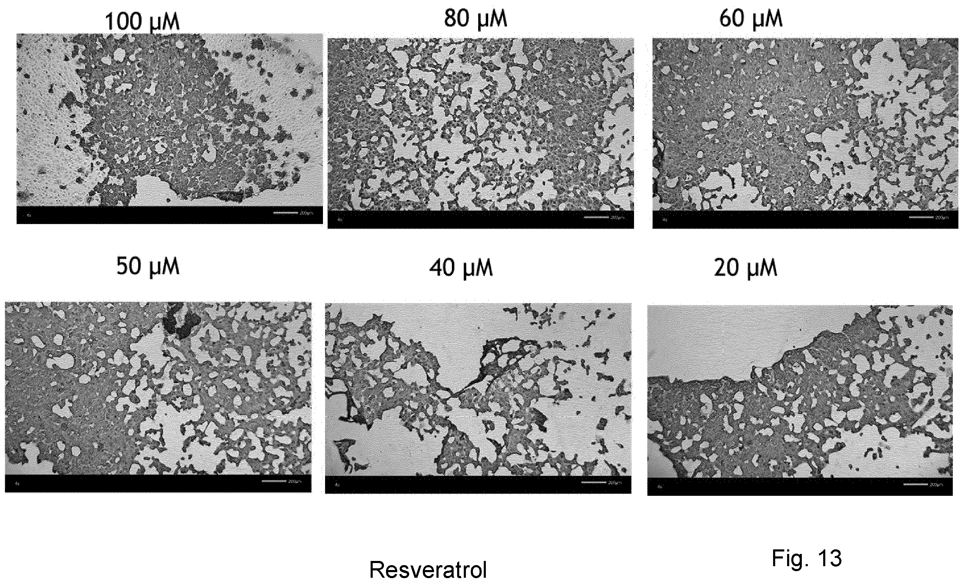

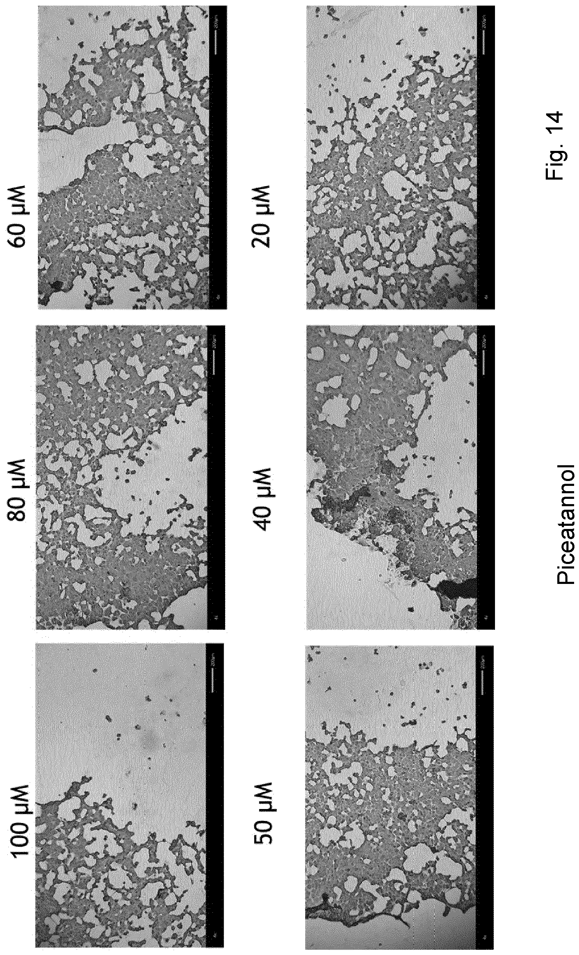

The results of these comparative tests are displayed in Figs. 9 to 14. Hexahydroxystilbene was able to inhibit viral replication completely (100 µM and 80 µM); trihydroxystilbene showed a low inhibition of viral replication (100 µM, shows concentration dependency); tetrahydroxystilbene showed no inhibition of viral replication.

## Claims

1. 3,3',4,4',5,5'-Hexahydroxy-trans-stilbene for use in the treatment and prevention of COVID-19 in a human subject, especially for inhibiting SARS-CoV-2.

2. 3,3',4,4',5,5'-Hexahydroxy-trans-stilbene for use according to claim 1, wherein inhibiting SARS-CoV-2 is inhibiting replication of SARS-CoV-2 and/or for preventing the cytopathic effect of an active virus replication of SARS-CoV-2 and/or preventing viral infections with SARS-CoV-2 through airborne channels.

3. A pharmaceutical preparation comprising or consisting of 3,3',4,4',5,5'-hexahydroxy-trans-stilbene as active ingredient and a pharmaceutically acceptable excipient for use according to claim 1 or 2.

4. A pharmaceutical preparation for use according to claim 3, wherein the preparation is for oral, sublingual, intramuscular, subcutaneous, nasal, oral mucosa, bronchial, pulmonary, skin, peritoneum or rectum administration, preferably for oral, sublingual or nasal administration.

5. A pharmaceutical preparation for use according to claim 3 or 4, wherein the preparation is provided as an inhalable and/or aerosol composition or as a nasal or oral spray and/or as sublingual films or tablets and/or as lollipops.

6. A pharmaceutical preparation for use according to any one of claims 3 to 5, wherein the pharmaceutically acceptable excipient is selected from diluents, such as water, acetone, ethanol, polyethylene glycol, polypropylene glycol, 1,3-propanediol, butane-1,3-diol, isopropyl myristate, isopropyl palmitate, mineral oil, ethoxylated isostearyl alcohol, polyoxylated isostearyl alcohol, polyoxyethylene sorbitol fatty acid ester; carrier, such as glycerol, mannitol, sorbitol, xylitol, and erythritol absorbents, such as starch, dextrin, calcium sulfate, lactose, mannitol, sucrose, sodium chloride, glucose, urea, kaolin, microcrystalline cellulose, aluminium silicate; wetting agents and binders, such as water, glycerin, polyethylene glycol, ethanol, propanol, starch syrup, dextrin, syrup, honey, glucose solution, acacia syrup, gelatin syrup, sodium carboxymethyl cellulose, purple Gum, methyl cellulose, potassium phosphate, polyvinylpyrrolidone; disintegrants, such as dried starch, alginate, agar powder, alginate, sodium bicarbonate, calcium carbonate, polyoxyethylene sorbitol fat acid esters, sodium lauryl sulfonate, methyl cellulose, ethyl cellulose; disintegration inhibitors, such as sucrose, glyceryl tristearate, cocoa butter, hydrogenated oil; absorption enhancers, such as quaternary ammonium salts, sodium lauryl sulfate; lubricants, such as talc, silicon dioxide, corn starch, stearic acid, boric acid, liquid paraffin, polyethylene glycol; polyacrylic resins, liposomes, water-soluble carriers such as PEG4000 and PEG6000, PVP; antioxidants; pH buffers and/or salt buffers; solubility modulators; penetration enhancers; antioxidants, such as citric acid and its salts, tartaric acid and its salts, phosphoric acid and its salts, and ethylenediaminetetraacetic acid (EDTA) and its salts, vitamins C, E; or mixtures thereof.

7. A pharmaceutical preparation for use according to any one of claims 3 to 6, wherein the preparation is an inhalation or aerosol preparation, especially an isotonic inhalation solution or suspension or a liquid or powdered aerosol.

8. A pharmaceutical preparation for use according to any one of claims 3 to 7, wherein the preparation contains 3,3',4,4', 5, 5'-hexahydroxy-trans-stilbene in an amount of from about 100 pg to about 10 g, preferably in an amount of from about 1 mg to about 1 g, more preferably in an amount of from 10 mg to 500 mg, even more preferably in an amount of from about 20 mg to about 300 mg.

9. A pharmaceutical preparation for use according to any one of claims 3 to 8, wherein the preparation contains 3,3',4,4',5,5'-Hexahydroxy-trans-stilbene in an amount of from about 10 mg to about 1000 mg, preferably in an amount of from about 25 mg to about 750 mg, more preferably in an amount of from 50 mg to 500 mg, even more preferably in an amount of from about 30 mg to about 100 mg.

10. A pharmaceutical preparation for use according to any one of claims 3 to 9, wherein the preparation is contained in a metered dose inhaler, in a dry powder inhaler, or in a nebulizer.

11. A pharmaceutical preparation for use according to any one of claims 3 to 9, wherein the preparation contains 3,3',4,4',5,5'-Hexahydroxy-trans-stilbene in 0.001% to 50% w/v, preferably in 0.01% to 20% w/v, more preferred in 0.1% to 20% w/v, especially in 1 % to 20% w/v.

## Patentansprüche

1. 3,3',4,4',5,5'-Hexahydroxy-trans-stilben zur Anwendung bei der Behandlung und Verhinderung von COVID-19 bei einem menschlichen Subjekt, insbesondere zur Hemmung von SARS-CoV-2.

2. 3,3',4,4',5,5'-Hexahydroxy-trans-stilben zur Anwendung gemäß Anspruch 1, wobei die Hemmung von SARS-CoV-2 die Hemmung der Replikation von SARS-CoV-2 und/oder die Verhinderung des zytopathischen Effekts einer aktiven Virusreplikation von SARS-CoV-2 und/oder die Verhinderung von Virusinfektionen mit SARS-CoV-2 über luftübertragene Kanäle ist.

3. Pharmazeutische Zubereitung, die 3,3',4,4',5,5'-Hexahydroxy-trans-stilben als Wirkstoff und einen pharmazeutisch annehmbaren Hilfsstoff enthält oder daraus besteht, zur Anwendung gemäß Anspruch 1 oder 2.

4. Pharmazeutische Zubereitung zur Anwendung gemäß Anspruch 3, wobei die Zubereitung zur oralen, sublingualen, intramuskulären, subkutanen, nasalen, oralen Schleimhaut-, bronchialen, pulmonalen, Haut-, Peritoneum- oder Rektalverabreichung, vorzugsweise zur oralen, sublingualen oder nasalen Verabreichung, vorgesehen ist.

5. Pharmazeutische Zubereitung zur Anwendung gemäß Anspruch 3 oder 4, wobei die Zubereitung als inhalierbare und/oder Aerosolzusammensetzung oder als Nasen- oder Mundspray und/oder als sublinguale Filme oder Tabletten und/oder als Lutscher bereitgestellt wird.

6. Pharmazeutische Zubereitung zur Anwendung gemäß einem der Ansprüche 3 bis 5, wobei der pharmazeutisch annehmbare Hilfsstoff ausgewählt ist aus Verdünnungsmitteln, wie Wasser, Aceton, Ethanol, Polyethylenglykol, Polypropylenglykol, 1,3-Propandiol, Butan-1,3-diol, Isopropylmyristat, Isopropylpalmitat , Mineralöl, ethoxylierter Isostearylalkohol, polyoxylierter Isostearylalkohol, Polyoxyethylensorbitfettsäureester; Trägerstoffe wie Glycerin, Mannit, Sorbit, Xylit und Erythrit; Absorptionsmittel wie Stärke, Dextrin, Calciumsulfat, Laktose, Mannit, Saccharose, Natriumchlorid, Glucose, Harnstoff, Kaolin, mikrokristalline Cellulose, Aluminiumsilikat; Benetzungsmittel und Bindemittel wie Wasser, Glycerin, Polyethylenglykol, Ethanol, Propanol, Stärkesirup, Dextrin, Sirup, Honig, Glukoselösung, Akaziensirup, Gelatinesirup, Natriumcarboxymethylcellulose, Purpur-Gummi, Methylcellulose, Kaliumphosphat , Polyvinylpyrrolidon; Sprengmittel wie getrocknete Stärke, Alginat, Agar-Pulver, Alginat, Natriumbicarbonat, Calciumcarbonat, PolyoxyethylensorbitFettsäureester, Natriumlaurylsulfonat, Methylcellulose, Ethylcellulose; Zerfallsinhibitoren wie Saccharose, Glyceryltristearat, Kakaobutter, gehärtetes Öl; Absorptionsverstärker wie quaternäre Ammoniumsalze, Natriumlaurylsulfat; Gleitmittel wie Talkum, Siliziumdioxid, Maisstärke, Stearinsäure, Borsäure, flüssiges Paraffin, Polyethylenglykol; Polyacrylharze, Liposomen, wasserlösliche Träger wie PEG4000 und PEG6000, PVP ; Antioxidantien; pH-Puffer und/oder Salzpuffer; Löslichkeitsmodulatoren; Penetrationsverstärker; Antioxidantien wie Zitronensäure und ihre Salze, Weinsäure und ihre Salze, Phosphorsäure und ihre Salze und Ethylendiamintetraessigsäure (EDTA) und ihre Salze, Vitamine C, E; oder Mischungen davon.

7. Pharmazeutische Zubereitung zur Anwendung gemäß einem der Ansprüche 3 bis 6, wobei die Zubereitung eine Inhalations- oder Aerosolzubereitung ist, insbesondere eine isotonische Inhalationslösung oder -suspension oder ein flüssiges oder pulverförmiges Aerosol.

8. Pharmazeutische Zubereitung zur Anwendung gemäß einem der Ansprüche 3 bis 7, wobei die Zubereitung 3,3',4,4',5,5'-Hexahydroxy-trans-stilben in einer Menge von etwa 100 pg bis etwa 10 g, vorzugsweise in einer Menge von etwa 1 mg bis etwa 1 g, mehr bevorzugt in einer Menge von 10 mg bis 500 mg, noch mehr bevorzugt in einer Menge von etwa 20 mg bis etwa 300 mg enthält.

9. Pharmazeutische Zubereitung zur Anwendung gemäß einem der Ansprüche 3 bis 8, wobei die Zubereitung 3,3',4,4',5,5'-Hexahydroxy-trans-stilben in einer Menge von etwa 10 mg bis etwa 1000 mg, vorzugsweise in einer Menge von etwa 25 mg bis etwa 750 mg, mehr bevorzugt in einer Menge von 50 mg bis 500 mg, noch mehr bevorzugt in einer Menge von etwa 30 mg bis etwa 100 mg enthält.

10. Pharmazeutische Zubereitung zur Anwendung gemäß einem der Ansprüche 3 bis 9, wobei die Zubereitung in einem Dosierinhalator, in einem Trockenpulverinhalator oder in einem Vernebler enthalten ist.

11. Pharmazeutische Zubereitung zur Anwendung gemäß einem der Ansprüche 3 bis 9, wobei die Zubereitung 3,3',4,4',5,5'-Hexahydroxy-trans-stilben in 0,001% bis 50% w/v, vorzugsweise in 0,01% bis 20% w/v, mehr bevorzugt in 0,1% bis 20% w/v, insbesondere in 1% bis 20% w/v enthält.

## Revendications

1. 3,3',4,4',5,5'-hexahydroxy-trans-stilbène destiné à être utilisé dans le traitement et la prévention de la COVID-19 chez un sujet humain, en particulier pour l'inhibition de SARS-CoV-2.

2. 3,3',4,4',5,5'-hexahydroxy-trans-stilbène destiné à être utilisé selon la revendication 1, où l'inhibition de SARS-CoV-2 est l'inhibition de la réplication de SARS-CoV-2 et/ou pour la prévention de l'effet cytopathique d'une réplication de virus actif de SARS-CoV-2 et/ou la prévention des infections virales par SARS-CoV-2 par des voies de transmission aériennes.

3. Préparation pharmaceutique comprenant ou consistant en 3,3',4,4',5,5'-hexahydroxy-trans-stilbène comme ingrédient actif et un excipient pharmaceutiquement acceptable destinée à être utilisée selon la revendication 1 ou 2.

4. Préparation pharmaceutique destinée à être utilisée selon la revendication 3, où la préparation est pour l'administration orale, sublinguale, intramusculaire, sous-cutanée, nasale, par voie muqueuse buccale, bronchique, pulmonaire, cutanée, par le péritoine ou le rectum, de préférence pour l'administration orale, sublinguale ou nasale.

5. Préparation pharmaceutique destinée à être utilisée selon la revendication 3 ou 4, où la préparation est fournie sous forme d'une composition inhalable et/ou en aérosol ou sous forme de spray nasal ou oral et/ou de films ou comprimés sublinguaux et/ou sous forme de sucettes.

6. Préparation pharmaceutique destinée à être utilisée selon l'une quelconque des revendications 3 à 5, où l'excipient pharmaceutiquement acceptable est choisi parmi les diluants, tels que l'eau, l'acétone, l'éthanol, le polyéthylèneglycol, le polypropylèneglycol, le 1,3-propanediol, le butane-1,3-diol, le myristate d'isopropyle, le palmitate d'isopropyle, l'huile minérale, l'alcool isostéarylique éthoxylé, l'alcool isostéarylique polyoxylé, un ester d'acide gras et de polyoxyéthylène sorbitol; un vecteur, tel que le glycérol, le mannitol, le sorbitol, le xylitol et l'érythritol, les agents absorbants tels que l'amidon, la dextrine, le sulfate de calcium, le lactose, le mannitol, le saccharose, le chlorure de sodium, le glucose, l'urée, le kaolin, la cellulose microcristalline, le silicate d'aluminium; les agents mouillants et les liants, tels que l'eau, la glycérine, le polyéthylèneglycol, l'éthanol, le propanol, le sirop d'amidon, la dextrine, le sirop, le miel, une solution de glucose, le sirop d'acacia, le sirop de gélatine, la carboxyméthylcellulose de sodium, la gomme pourpre, la méthylcellulose, le phosphate de potassium, la polyvinylpyrrolidone; les désintégrants, tels que l'amidon séché, l'alginate, la poudre d'agar, l'alginate, le bicarbonate de sodium, le carbonate de calcium, les esters d'acide gras et de polyoxyéthylènesorbitol, le laurylsulfonate de sodium, la méthylcellulose, l'éthylcellulose; les inhibiteurs de désintégration, tels que le saccharose, le tristéarate de glycéryle, le beurre de cacao, l'huile hydrogénée; les activateurs d'absorption, tels que les sels d'ammonium quaternaire, le laurylsulfate de sodium; les lubrifiants, tels que le talc, le dioxyde de silicium, l'amidon de maïs, l'acide stéarique, l'acide borique, la paraffine liquide, le polyéthylèneglycol; les résines polyacryliques, les liposomes, les vecteurs hydrosolubles tels que le PEG4000 et le PEG6000, la PVP; les antioxydants; les tampons de pH et/ou les tampons de sel; les modulateurs de solubilité; les activateurs de pénétration; les antioxydants, tels que l'acide citrique et ses sels, l'acide tartrique et ses sels, l'acide phosphorique et ses sels, et l'acide éthylènediaminetétraacétique (EDTA) et ses sels, les vitamines C, E; ou leurs mélanges.

7. Préparation pharmaceutique destinée à être utilisée selon l'une quelconque des revendications 3 à 6, où la préparation est une préparation pour inhalation ou en aérosol, en particulier une solution ou une suspension isotonique pour inhalation ou un aérosol liquide ou en poudre.

8. Préparation pharmaceutique destinée à être utilisée selon l'une quelconque des revendications 3 à 7, où la préparation contient du 3,3',4,4',5,5'-hexahydroxy-trans-stilbène en une quantité d'environ 100 µg à environ 10 g, de préférence en une quantité d'environ 1 mg à environ 1 g, de préférence encore en une quantité d'environ 10 mg à 500 mg, de préférence encore en une quantité d'environ 20 mg à environ 300 mg.

9. Préparation pharmaceutique destinée à être utilisée selon l'une quelconque des revendications 3 à 8, où la préparation contient du 3,3',4,4',5,5'-hexahydroxy-trans-stilbène en une quantité d'environ 10 mg à environ 1000 mg, de préférence en une quantité d'environ 25 mg à environ 750 mg, de préférence encore en une quantité d'environ 50 mg à 500 mg, de préférence encore en une quantité d'environ 30 mg à environ 100 mg.

10. Préparation pharmaceutique destinée à être utilisée selon l'une quelconque des revendications 3 à 9, où la préparation est contenue dans un inhalateur doseur, dans un inhalateur à poudre sèche ou dans un nébuliseur.

11. Préparation pharmaceutique destinée à être utilisée selon l'une quelconque des revendications 3 à 9, où la préparation contient du 3,3',4,4',5,5'-hexahydro>cy-trans-stilbène à raison de 0,001 % à 50 % plv, de préférence à raison de 0,01 % à 20 % plv, de préférence encore à raison de 0,1 % à 20 % p/v, en particulier à raison de 1 % à 20 % plv.
